# EUROPÄISCHE PATENTANMELDUNG

(11) **EP 4 574 188 A1**
(43) Veröffentlichungstag der Anmeldung: **25.06.2025**
(21) Anmeldenummer: 23219998.4
(22) Anmeldetag: 22.12.2023
(51) Int. Cl.: A61M 5/315, A61M 5/00, A61M 5/178, A61M 5/50, A61M 5/31

(54) **MEDIZINISCHES APPLIKATIONSGEFÄSS MIT EINEM VORFÜLLBAREN SPRITZENKÖRPER**

(71) Anmelder: Transcoject GmbH, 24539 Neumünster (DE)
(72) Erfinder: Heinz, Jochen, 24220 Flintbek (DE)
(74) Vertreter: Patentanwälte Hemmer Lindfeld Frese Partnerschaft mbB

(57) **Zusammenfassung**

Die vorliegende Offenbarung betrifft ein medizinisches Applikationsgefäß (1) mit einem mit einer Substanz zumindest teilweise vorfüllbaren Spritzenkörper (3) zum Injizieren der Substanz in ein Lebewesen, wobei sich der Spritzenkörper (3) von einem proximalen Ende (5) des Spritzenkörpers (3) zu einem distalen Ende (7) des Spritzenkörpers (3) erstreckt und ein erstes Innenvolumen (9) definiert, in welches die Substanz vorfüllbar ist, wobei am distalen Ende (7) des Spritzenkörpers (3) eine Injektionsnadel (57) oder ein Injektionsschlauch (63) angeschlossen oder anschließbar ist, wobei das erste Innenvolumen (9) proximalseitig durch einen auf das distale Ende (7) zu bewegbaren Stopfen (13) begrenzt ist, wobei der Stopfen (13) proximalseitig eine Kolbenstangenaufnahme (15) für eine vom Stopfen (13) separate Kolbenstange (17) aufweist, wobei proximal vom Stopfen (13) ein manuell entfernbares und/oder mittels der Kolbenstange (17) durchstoßbares proximales Dichtmittel (21) angeordnet ist, welches ein axial zwischen dem proximalen Dichtmittel (21) und dem Stopfen (13) ausgebildetes zweites Innenvolumen (19) proximalseitig abdichtet.

## Beschreibung

Die vorliegende Offenbarung betrifft ein medizinisches Applikationsgefäß mit einem mit einer Substanz zumindest teilweise vorfüllbaren Spritzenkörper zum Injizieren der Substanz in ein Lebewesen. Es geht also um vorfüllbare oder vorgefüllte medizinische Spritzensysteme.

Vorgefüllte medizinische Spritzen haben den Vorteil, dass medizinisches Personal die Spritzen vor der Injektion nicht mit der zu injizierenden Substanz aus einer Ampulle aufziehen müssen, sondern anwendungsbereit dem medizinischen Personal zur Verfügung stehen. Vorgefüllte Spritzen werden also dem medizinischen Personal bereits vorgefüllt geliefert. Solche Spritzen sind beispielsweise in der ISO 11040 beschrieben. Aus der EP 2 900 301 B1 ist beispielsweise ebenso eine vorgefüllte Spritze bekannt. Eine technische Herausforderung für vorgefüllte Spritzen ist allerdings, dass sie Medikamente und Medizinprodukte sicher über einen längeren Zeitraum aufbewahren müssen. Es sollte eine Aufbewahrungszeit von mindestens drei Jahren garantiert werden können, innerhalb welcher die enthaltene Substanz sicher in der vorgefüllten Spritze aufbewahrt wird. Dabei hängt die mögliche Aufbewahrungszeit einer Substanz wesentlich von den Barriereeigenschaften der Spritze, der für Spritzenkörper und Verschluss verwendeten Materialien und der Empfindlichkeit sowie der Menge der in der Spritze enthaltenen Substanz ab. Bei Verlust von Wasserdampf oder Lösungsmittelflüssigkeit kann sich beispielsweise die Konzentration eines medizinischen Wirkstoffs innerhalb der Substanz verändern. Je kleiner und empfindlicher die Substanzmenge ist, umso weniger tolerant kann man gegenüber ausdiffundierenden Verlusten oder eindiffundierenden Verunreinigungen sein. Es könnte sich beispielsweise durch Migration von Kohlendioxid in das Spritzenmaterial der pH-Wert der Substanz verändern. Schließlich könnte eindringender Sauerstoff einen medizinischen Wirkstoff schädigen.

Daher ist es für vorgefüllte Spritzensysteme im Allgemeinen und für Spritzensysteme aus Kunststoff im Besonderen sehr wichtig, dass diese mit Elastomerkomponenten ausgestattet sind, die gute Barriereeigenschaften aufweisen. Problematisch bei vielen Elastomerkomponenten mit guten Barriereeigenschaften ist allerdings, dass sie mit vielen Kunststoffen interagieren und damit zu sehr hohen Öffnungskräften und/oder Reibkräften bei der Applikation der Spritzen führen. Andere Elastomermaterialien besitzen keine ausreichende Reinheit, sind nicht biokompatibel oder enthalten extrahierbare Substanzen, die das in der Spritze enthaltene Füllgut verunreinigen können.

Es ist daher die Aufgabe der vorliegenden Offenbarung ein biokompatibles medizinisches Applikationsgefäß mit verbesserten Barriereeigenschaften bereitzustellen. Die Barriereeigenschaften sollen so weit verbessert werden, dass das medizinische Applikationsgefäß mit Substanzen vorfüllbar ist, die bisher nicht in vorgefüllten Spritzen gelagert werden konnten, insbesondere nicht in kleinen Mengen.

Gelöst wird diese Aufgabe durch ein medizinisches Applikationsgefäß gemäß Anspruch 1. Bevorzugte Ausführungsformen der Erfindung können den Unteransprüchen, der Beschreibung und den Figuren entnommen werden.

Erfindungsgemäß wird ein medizinisches Applikationsgefäß bereitgestellt mit einem mit einer Substanz zumindest teilweise vorfüllbaren Spritzenkörper zum Injizieren der Substanz in ein Lebewesen, wobei sich der Spritzenkörper von einem proximalen Ende des Spritzenkörpers zu einem distalen Ende des Spritzenkörpers erstreckt und ein erstes Innenvolumen definiert, in welches die Substanz vorfüllbar ist, wobei am distalen Ende des Spritzenkörpers eine Injektionsnadel oder ein Injektionsschlauch angeschlossen oder anschließbar ist, wobei das erste Innenvolumen proximalseitig durch einen auf das distale Ende zu bewegbaren Stopfen begrenzt ist, wobei der Stopfen proximalseitig eine Kolbenstangenaufnahme für eine vom Stopfen separate Kolbenstange aufweist, wobei proximal vom Stopfen ein manuell entfernbares und/oder mittels der Kolbenstange durchstoßbares proximales Dichtmittel angeordnet ist, welches ein axial zwischen dem proximalen Dichtmittel und dem Stopfen ausgebildetes zweites Innenvolumen proximalseitig abdichtet.

Es sei an dieser Stelle angemerkt, dass sich die Begriffe "distal" und "proximal" auf die das Applikationsgefäß handhabende Person, also das medizinische Personal, bezieht und nicht auf das Lebewesen, dem die Substanz injiziert wird. Die Erfindungsidee ist hier also, die Substanz in einem ersten Innenvolumen aufzubewahren und proximal vom Stopfen ein zweites Innenvolumen vorzusehen, welches durch das proximale Dichtmittel abgedichtet ist. Dadurch ist die Substanz proximalseitig sowohl durch den Stopfen als auch durch das proximale Dichtmittel von der Umgebung getrennt, sodass sowohl der Stopfen als auch das proximale Dichtmittel in Summe eine proximalseitige Barriere darstellen. Der Vorteil der Erfindung liegt darin, dass der Stopfen aus einem Material mit weniger Barrierewirkung ausgebildet werden kann, das aber biokompatibel mit Substanzen ist, die bisher nicht in vorgefüllten Spritzen gelagert werden konnten. Das proximale Dichtmittel muss nicht gleitfähig im Spritzenkörper beweglich sein, sodass dafür ein Material mit besseren Barriereeigenschaften verwendet werden kann. Das Zusammenspiel von Stopfen und proximalem Dichtmittel erhöht also nicht nur insgesamt die Barrierewirkung in proximale Richtung, sondern schafft zudem die Möglichkeit, verschiedene Materialien für den Stopfen und das proximale Dichtmittel zu verwenden, um den jeweiligen Anforderungen gerecht zu werden. Beispielsweise kann für den Stopfen ein Flüssigsilikonkautschuk (liquid silicone rubber, LSR) eingesetzt werden, sodass kein Silikonöl für die Schmierung des Stopfens benötigt wird. Bestimmte Substanzen, insbesondere in kleiner Menge, dürfen nämlich selbst mit kleinsten Mengen Silikonöl nicht verunreinigt werden. Eine mangelnde Barrierewirkung des Flüssigsilikonkautschuks (LSR) kann durch das proximale Dichtmittel kompensiert oder überkompensiert werden, das beispielsweise ein artgleiches oder artähnliches Material wie der Spritzenkörper aufweisen kann, z.B. ein Cyclo-Olefin-Polymer (COP). Alternativ oder zusätzlich kann das proximale Dichtmittel eine Aluminiumschicht aufweisen oder allgemein eine metallisierte Folie.

Es sei an dieser Stelle angemerkt, dass die vorliegende Erfindung nur medizinische Applikationsgefäße betrifft, die einen vorfüllbaren oder vorgefüllten Spritzenkörper aufweisen. Applikationsgefäße, die vom medizinischen Personal selbst mit der Substanz aus einer Ampulle aufgezogen werden müssen, sind nicht von der vorliegenden Erfindung erfasst. Ist hierin also von einem "vorfüllbaren" Spritzenkörper die Rede, so ist damit ein Spritzenkörper gemeint, der dazu geeignet und bestimmt ist, mit einer Substanz zumindest teilweise vorgefüllt zu werden. Beim Herstellungsprozess von vorgefüllten medizinischen Applikationsgefäßen ist es nämlich oft so, dass ein Herstellerunternehmen der Applikationsgefäße diese unbefüllt an ein Unternehmen liefert, welches die Applikationsgefäße mit der Substanz vorbefüllt und dann als vorgefülltes Applikationsgefäß an das medizinische Personal liefert. Die Befüllung des Spritzenkörpers mit der Substanz kann von proximaler Seite oder von distaler Seite des Spritzenkörpers erfolgen.

Optional kann der Spritzenkörper zumindest teilweise mit der Substanz vorgefüllt sein, sodass sich die Substanz im ersten Innenvolumen befindet. In diesem Fall richtet sich die Erfindung auf das Produkt, wie es dem medizinischen Personal geliefert wird.

Optional kann das Applikationsgefäß eine proximalwärts weisende Ringfläche aufweisen, auf welche das proximale Dichtmittel in Form einer manuell abziehbaren und/oder mittels der Kolbenstange durchstoßbaren Dichtfolie dichtend aufgebracht ist. Die Kosten einer Dichtfolie und das Aufbringen auf einer proximalwärts weisenden Ringfläche ist besonders einfach und günstig im Gegensatz zu hochwertigen Stopfenbeschichtungen oder alternativen Dichtmitteln.

Optional kann am distalen Ende des Spritzenkörpers ein Anschluss für die Injektionsnadel oder den Injektionsschlauch in Form eines Luer- oder Luer-Lock-Anschlusses ausgebildet sein. Dies ist sinnvoll, damit das medizinische Applikationsgefäß mit einer Vielzahl von üblichen Injektionsnadeln oder Injektionsschläuchen über einen standardisierten Luer- oder Luer-Lock-Anschlusses verbunden werden kann. Typischerweise ist der Luer- oder Luer-Lock-Anschluss am Applikationsgefäß männlich und der dazu passende Luer- oder Luer-Lock-Anschluss der Injektionsnadel oder des Injektionsschlauchs weiblich. Andere normativ beschriebene oder auch individuell angepasste Anschlüsse sind ebenso möglich

Optional kann der Spritzenkörper distalseitig mit einem distalen Dichtmittel verschließbar oder verschlossen sein, wobei das distale Dichtmittel mittels der Injektionsnadel oder des Injektionsschlauchs durchstoßbar ist. Dies ist sinnvoll, um auch an der distalen Seite des Spritzenkörpers eine verbesserte Barrierewirkung zu erzielen. Das distale Dichtmittel kann das erste Innenvolumen distalseitig verschließen, sodass das distale Dichtmittel in direktem Kontakt mit der vorgefüllten Substanz steht. Dazu kann das distale Dichtmittel eine proximal weisende Oberfläche aufweisen, die biokompatibel mit der vorgefüllten Substanz ist. Das distale Dichtmittel kann in Form einer Folie auf eine distale Stirnfläche des Luer- oder Luer-Lock-Anschlusses des Applikationsgefäßes aufgebracht sein. Analog zum proximalen Dichtmittel kann das distale Dichtmittel proximalseitig mit einer Schicht beschichtet sein, die das erste Innenvolumen distalseitig begrenzt, wobei die Schicht aus dem gleichen Material ist wie der Spritzenkörper, vorzugsweise aus einem Cyclo-Olefin-Polymer (COP) oder einem Polypropylen (PP).

Optional kann das proximale Dichtmittel distalseitig mit einer Schicht beschichtet sein, die das zweite Innenvolumen proximalseitig begrenzt, wobei die Schicht aus dem gleichen Material ist wie der Spritzenkörper, vorzugsweise aus einem Cyclo-Olefin-Polymer (COP) oder einem Polypropylen (PP). Hierdurch ist eine Biokompatibilität mit der Substanz gewährleistet für den Fall, dass Teile der Substanz durch den Stopfen in das zweite Innenvolumen diffundieren.

Optional kann der Spritzenkörper aus Glas sein und/oder einen Kunststoff aufweisen, vorzugsweise ein Cyclo-Olefin-Polymer (COP) oder Polypropylen (PP). Glas hat eine hervorragende Barrierewirkung und ist biokompatibel, allerdings einerseits nicht so günstig herstellbar und andererseits auch nicht so maßgenau herstellbar wie ein Spritzenkörper aus Kunststoff, insbesondere bei großen Spritzenvolumina. Mit einem Cyclo-Olefin-Polymer (COP) oder einem Polypropylen (PP) lässt sich günstig und maßgenau ein Spritzenkörper auch mit kleinem Volumen aus Kunststoff herstellen, der sowohl biokompatibel ist als auch eine hinreichende Barrierewirkung hat.

Optional kann das proximale Dichtmittel mit einer proximalwärts weisenden Ringfläche des Applikationsgefäßes verschweißt und/oder stoffschlüssig verbunden und/oder kraftschlüssig und/oder formschlüssig verbunden sein. Dies ist vorteilhaft, um das zweite Innenvolumen sicher mit dem proximalen Dichtmittel abzudichten. Hier kann beispielsweise ein Schmelzkleber oder ein anderer Kleber verwendet werden, um das proximale Dichtmittel aufzukleben. Zusätzlich oder alternativ kann beispielsweise eine kraftschlüssige und/oder formschlüssige Verbindung durch ein Einprägen des proximalen Dichtmittels in die proximalwärts weisende Ringfläche erzielt werden.

Optional kann das proximale Dichtmittel aus einer Aluminiumschicht oder einem Laminat mit einer Aluminiumschicht bestehen. Vorzugsweise besteht das proximale Dichtmittel aus einem Laminat mit einer Aluminiumschicht und/oder einer Schicht aus einem Cyclo-Olefin-Polymer (COP), wobei die Schicht aus dem Cyclo-Olefin-Polymer (COP) vorzugsweise an das zweite Innenvolumen angrenzt. Durch die Aluminiumschicht kann eine besonders hohe Barrierewirkung erzielt werden und durch die COP-Schicht kann eine Biokompatibilität erzielt werden.

Optional kann das proximale Dichtmittel mittels einer am proximalen Ende des Spritzenkörpers montierten Griffhilfe und/oder eines Backstopps unter federnder Vorspannung auf eine proximalwärts weisende Ringfläche des Applikationsgefäßes gedrückt sein. Dadurch ist das proximale Dichtmittel besonders sicher gegen äußere Einflüsse mechanisch fixiert. Bei dieser Ausführungsform ist es vorteilhaft, wenn das proximale Dichtmittel nicht manuell entfernt wird, sondern mittels der Kolbenstange durchstoßen wird. Dazu weist die montierte Griffhilfe und/oder der Backstopp vorzugsweise eine zentrale Öffnung auf, um die Kolbenstange hindurchstoßen zu können.

Optional kann das axial zwischen dem proximalen Dichtmittel und dem Stopfen angeordnete zweite Innenvolumen mit einem anderen Gas als Luft gefüllt sein. Dies kann beispielsweise sinnvoll sein, wenn eine sauerstoffempfindliche Substanz vorgefüllt ist oder wird, welche bereits bei einem Sauerstoffgehalt im zweiten Innenvolumen Schaden nehmen könnte, wenn Sauerstoff daraus durch den Stopfen in das erste Innenvolumen diffundiert. Das zweite Innenvolumen ist daher vorzugsweise mit einem sauerstoffreduzierten oder völlig sauerstofflosen Gas gefüllt. Alternativ oder zusätzlich kann das zweite Innenvolumen ein Vakuum bzw. einen Unterdruck aufweisen.

Optional kann das proximale Dichtmittel proximalseitig mit Daten einer Substanzbefüllung und/oder einer einmaligen Produktkennung (unique device identification, UDI) bedruckt sein. Die Daten können beispielsweise die Art, Menge und/oder Bestandteile der Substanz beinhalten. Vorzugsweise beinhalten die Daten ein Befüllungsdatum und/oder ein Mindesthaltbarkeitsdatum. Die Daten und/oder die UDI können alphanummerisch lesbar und/oder kodiert maschinenlesbar, beispielsweise als Strichcode oder QR-Code, aufgedruckt sein.

Optional kann das proximale Dichtmittel als von außen sichtbares Qualitätsegel dienen. Für einen Anwender ist dadurch sofort erkennbar, ob das proximale Dichtmittel selbst und/oder die dichtende Verbindung mit dem Spritzenkörper beschädigt ist.

Optional kann der Stopfen zumindest umfangseitig einen Flüssigsilikonkautschuk (liquid silicone resin, LSR) aufweisen, der in gleitfähigem Kontakt mit dem Spritzenkörper steht und vorzugsweise bei Temperaturen oberhalb von -100°C elastisch ist. Der Stopfen ist dadurch besonders gleitfähig und kann ohne Silikonöl verwendet werden, welches für bestimmte Substanzen insbesondere kleiner Mengen problematisch wäre. Eine etwaige mangelnde Barrierewirkung des LSR kann durch die zusätzliche Barrierewirkung des proximalen Dichtmittels kompensiert bzw. überkompensiert werden.

Optional kann der Stopfensilikonöl frei und gleit beschichtungsfrei in gleitfähigem Kontakt mit dem Spritzenkörper stehen. Dies ist insbesondere für kleinvolumige Applikationsgefäße vorteilhaft, die z.B. in der Augenheilkunde eingesetzt werden, bei denen selbst kleinste Mengen von Silikonöl oder einem anderen Gleitmittel schädlichen Einfluss auf die Substanz hätten, oder z.B. im Auge des Patienten dauerhaft zu sehen wären.

Optional kann der Spritzenkörper einstückig ausgebildet sein und eine proximalseitige Stirnseite des Spritzenkörpers eine proximalwärts weisende Ringfläche bilden, auf welche das proximale Dichtmittel in Form einer manuell abziehbaren und/oder mittels der Kolbenstange durchstoßbare Dichtfolie dichtend aufgebracht ist. Dies ist eine besonders einfache Ausführungsform des erfindungsgemäßen Applikationsgefäßes, wobei der Spritzenkörper vorzugsweise einstückig gegossen ist.

Optional kann der Spritzenkörper aus mindestens zwei miteinander verbindbaren oder verbundenen Spritzenkörperteilen ausgebildet sein, wobei die Spritzenkörperteile mindestens einen distalen Spritzenkörperteil und einen proximalen Spritzenkörperteil aufweisen, wobei das erste Volumen im Wesentlichen vom distalen Spritzenkörperteil umschlossen ist und das zweite Innenvolumen im Wesentlichen vom proximalen Spritzenkörperteil umschlossen ist. Der distale Spritzenkörperteil kann einen Hauptteil des Spritzenkörpers bilden und der proximale Spritzenkörperteil einen Kopfteil des Spritzenkörpers. Die beiden Spritzenkörperteile weisen vorzugsweise das gleiche Material auf, können allerdings auch verschiedene Materialien aufweisen. Die Spritzenkörperteile können miteinander verschweißt und/oder stoffschlüssig verbunden und/oder kraftschlüssig und/oder formschlüssig verbunden sein. Beispielsweise können sie miteinander verschraubt und/oder verklebt sein.

Optional kann der proximale Spritzenkörperteil mit dem proximalen Dichtmittel eine vormontierte Einheit bilden, bei der das proximale Dichtmittel dichtend auf einer proximalwärts weisenden Ringfläche des proximalen Spritzenkörperteils sitzt, wobei die vormontierte Einheit mit dem mit der Substanz befüllten distalen Spritzenkörperteil verbindbar oder verbunden ist. Dies kann für den Herstellungsprozess vorteilhaft sein, wenn es einfacher ist, die vormontierte Einheit mit dem befüllten distalen Spritzenkörperteil zu verbinden als das proximale Dichtmittel auf dem befüllten Spritzenkörper aufzubringen. Die vormontierte Einheit kann dann außerhalb der Befüllungsumgebung unter weniger strengen Umgebungsvoraussetzungen vormontiert werden.

Optional kann der distale Spritzenkörperteil axial länger sein als der proximale Spritzenkörperteil, vorzugsweise um ein Vielfaches. Dies ist sinnvoll, um das Applikationsgefäß nicht länger als nötig auszugestalten, da das zweite Innenvolumen sehr viel kleiner ausgestaltet werden kann als das erste Innenvolumen, dessen Größe im Wesentlichen dadurch bestimmt ist, welche Menge an Substanz in dem Applikationsgefäß vorgefüllt sein soll.

Im Folgenden wird die Erfindung detaillierter anhand der beiliegenden Figuren erläutert. Es zeigen:
- Fig. 1: eine Längsansicht eines Ausführungsbeispiels eines erfindungsgemäßen Applikationsgefäßes mit einer Detail-Längsschnittansicht auf einen proximalen Abschnitt des Applikationsgefäßes;
- Fig. 2: das Applikationsgefäß aus Fig. 1 beim Durchstoßen des proximalen Dichtmittels mit der Kolbenstange;
- Fig. 3a, b: wie das proximale Dichtmittel des Applikationsgefäßes aus Fig. 1 manuell abziehbar ist.
- Fig. 4: eine Ausführungsform des erfindungsgemäßen Applikationsgefäßes mit einer Detailansicht wie das distale Dichtmittel dichtend aufgebracht ist;
- Fig. 5: eine Ausführungsform des erfindungsgemäßen Applikationsgefäßes mit einem proximalen Dichtmittel in Form einer Laminatfolie;
- Fig. 6: eine Ausführungsform des erfindungsgemäßen Applikationsgefäßes mit einer Griffhilfe;
- Fig. 7a-c: eine Ausführungsform des erfindungsgemäßen Applikationsgefäßes mit einer anderen Kolbenstangenausführung und zugehörigen Stopfenausführung;
- Fig. 8: eine Ausführungsform des erfindungsgemäßen Applikationsgefäßes mit zweiteiligem Spritzenkörper, wobei die Spritzenkörperteile miteinander verklebt sind;
- Fig. 9: eine Ausführungsform des erfindungsgemäßen Applikationsgefäßes mit zweiteiligem Spritzenkörper, wobei die Spritzenkörperteile miteinander verschraubt sind;
- Fig. 10a-b: eine Ausführungsform des erfindungsgemäßen Applikationsgefäßes mit distalem Dichtmittel; und
- Fig. 11a,: b eine Injektionsnadel und ein Injektionsschlauch, welche an eine Ausführungsform des erfindungsgemäßen Applikationsgefäßes anschließbar sind.

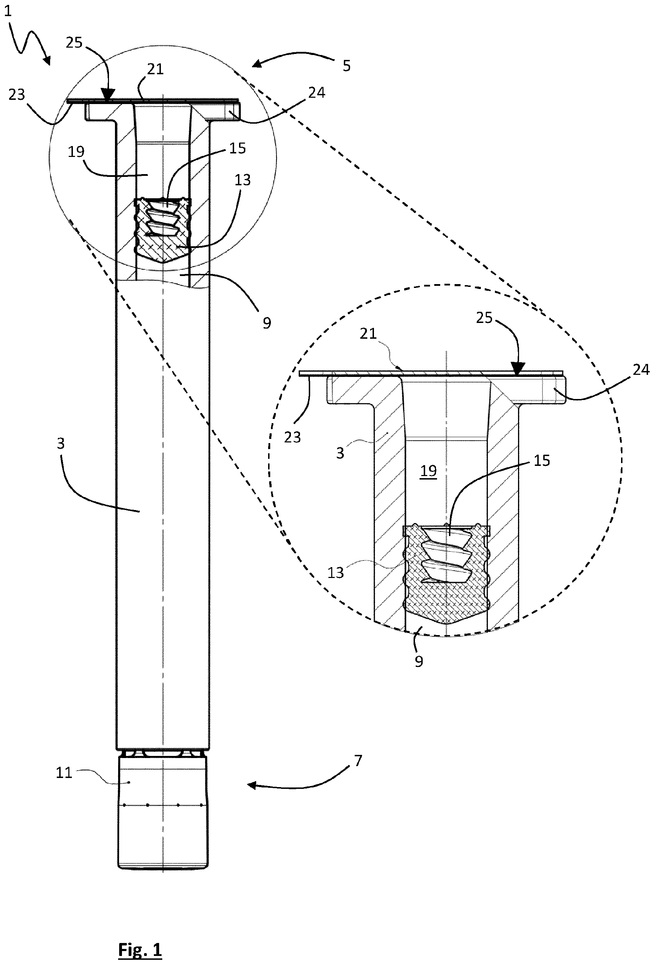

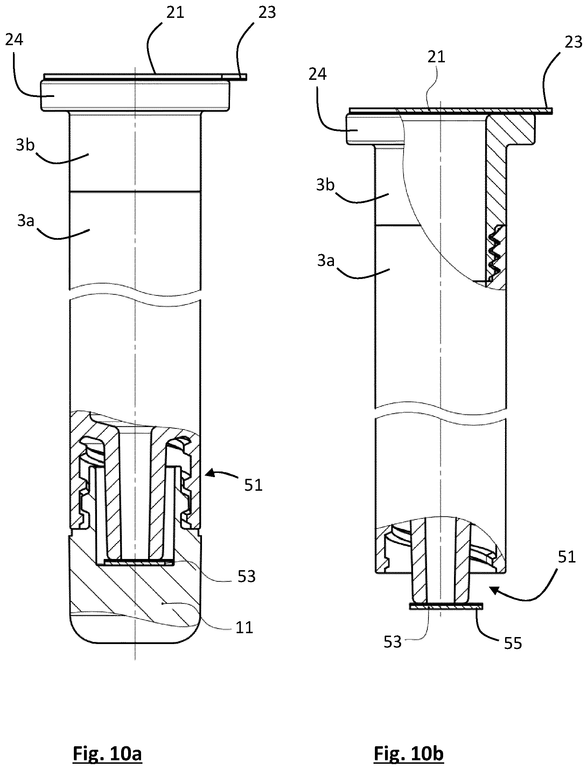

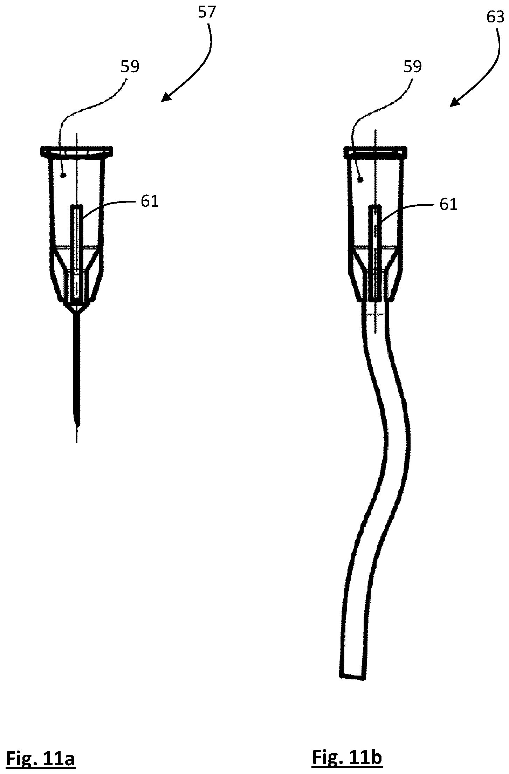

Fig. 1 zeigt ein Ausführungsbeispiel eines erfindungsgemäßen medizinischen Applikationsgefäßes 1, das einen Spritzenkörper 3 aufweist, der dazu geeignet und bestimmt ist, mit einer Substanz zumindest teilweise vorgefüllt zu werden, um die Substanz in ein Lebewesen, beispielsweise einen Patienten, zu injizieren. Die Substanz kann beispielsweise ein fließfähiges Medium mit einem medizinischen Wirkstoff sein. Der Spritzenkörper 3 erstreckt sich von einem proximalen Ende 5 des Spritzenkörpers 3 des Applikationsgefäßes 1 zu einem distalen Ende 7 des Spritzenkörpers 3 des Applikationsgefäßes 1 und definiert ein erstes Innenvolumen 9, in welches die Substanz vorfüllbar ist. Das proximale Ende 5 ist in den Figuren jeweils oben und das distale Ende 7 jeweils unten angeordnet. Es sei an dieser Stelle angemerkt, dass sich die Begriffe "distal" und "proximal" auf die Anwendungsperson des Applikationsgefäßes 1 beziehen und nicht auf das Lebewesen, in welches die vorgefüllte Substanz injiziert wird. Das Applikationsgefäß 1 wird also von einer Anwendungsperson am proximalen Ende 5 des Spritzenkörpers 3 gehalten, und am distalen Ende 7 des Spritzenkörpers 3 ist eine Injektionsnadel oder ein Injektionsschlauch (s. Fig. 11a,b) anschließbar. Zum Anschluss einer Injektionsnadel oder eines Injektionsschlauchs an das Applikationsgefäß 1 wird in dem in Fig. 1 gezeigten Ausführungsbeispiel zunächst eine distalseitige Kappe 11 abgenommen, um einen distalseitigen Luer- oder Luer-Lock-Anschluss (s. Fig. 10a,b) freizugeben.

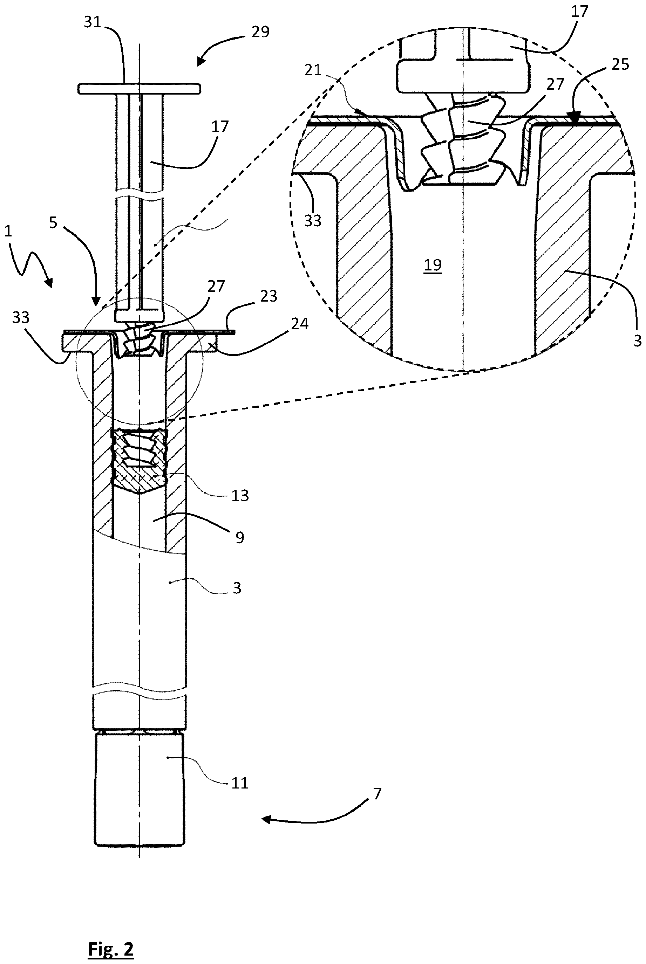

Der Spritzenkörper 3 des Applikationsgefäßes 1 ist in dem in Fig. 1 gezeigten Ausführungsbeispiel einstückig aus einem Kunststoff geformt, der ein Ccklo-Olefin-Polymer (COP) aufweist. Das vom Spritzenkörper 3 des Applikationsgefäßes 1 definierte erste Innenvolumen 9 hat hier die Form eines länglichen Zylinders mit vorzugsweise kreisrundem Querschnitt. Proximalseitig, also in Fig. 1 von oben, ist das erste Innenvolumen 9 durch einen auf das distale Ende 7 zu bewegbaren Stopfen 13 begrenzt. Der Stopfen 13 weist zumindest umfangseitig einen Flüssigsilikonkautschuk (liquid silicone resin, LSR) auf, der silikonölfrei und gleitbeschichtungsfrei in gleitfähigem Kontakt mit dem Spritzenkörper 3 steht und vorzugsweise bei Temperaturen oberhalb von -100°C elastisch ist. Der Stopfen 13 weist proximalseitig, also in Fig. 1 auf seiner Oberseite, eine Kolbenstangenaufnahme 15 zur Aufnahme einer vom Stopfen 13 separaten Kolbenstange 17 (s. Fig. 2) auf. Die Kolbenstangenaufnahme 15 ist hier als ein relativ grobes Innengewinde mit wenigen Windungen ausgebildet.

Proximal vom Stopfen 13, also in Fig. 1 über dem Stopfen 13, befindet sich ein zweites Innenvolumen 19. Das zweite Innenvolumen 19 ist sehr viel kürzer als das erste Innenvolumen 9 und ist nicht mit der Substanz vorgefüllt. Das zweite Innenvolumen 19 kann mit Luft oder vorzugsweise mit einem anderen Gas als Luft gefüllt sein. Distalseitig, also in Fig. 1 von unten, dichtet der Stopfen 13 das zweite Innenvolumen 19 ab und trennt somit das zweite Innenvolumen 19 vom ersten Innenvolumen 9. Proximalseitig, also in Fig. 1 von oben, ist das zweite Innenvolumen 19 durch ein proximales Dichtmittel 21 abgedichtet. Das proximale Dichtmittel 21 ist manuell entfernbar und/oder mittels der Kolbenstange 17 durchstoßbar. In dem in Fig. 1 gezeigten Ausführungsbeispiel ist das proximale Dichtmittel 21 eine manuell abziehbare Dichtfolie, welche zumindest distalseitig, also zum zweiten Innenvolumen 19 hin, eine Schicht aus dem gleichen Material wie der Spritzenkörper 3 aufweist, also aus einem Cyclo-Olefin-Polymer (COP). Um die manuelle Abziehbarkeit der Dichtfolie 21 zu erleichtern, steht die Dichtfolie 21 lateral an zumindest einer Seite mit einer Grifflasche 23 über, um von einer Anwendungsperson mit einem Pinzettengriff von Daumen- und Zeigefinger einfach gegriffen werden zu können und die Dichtfolie 21 abzuziehen. Der Spritzenkörper 3 weist an seinem proximalen Ende 5 einen Kragen 24 auf, der eine proximalwärts weisende Ringfläche 25 ausbildet. Die Dichtfolie 21 ist hier mit der Ringfläche 25 umlaufend dichtend verschweißt oder verklebt.

In Fig. 2 ist gezeigt, wie das proximale Dichtmittel 21 mit einer Kolbenstange 17 durchstoßen wird. Das Durchstoßen des proximalen Dichtmittels mit der Kolbenstange 17 kann zusätzlich zum manuellen Entfernen des proximalen Dichtmittels 21 vorgesehen sein oder alternativ dazu. Die Kolbenstange 17 kann zusammen mit dem medizinischen Applikationsgefäß 1 als separates Zubehörteil mitgeliefert werden oder bereits im Besitz der Anwendungsperson sein. Die Kolbenstange 17 kann ggf. für mehrere Applikationsgefäße 1 wiederverwendet werden. Die Kolbenstange 17 muss allerdings zum Applikationsgefäß 1 passen, d.h. entsprechend lang ausgebildet sein, um den Stopfen 13 ganz bis an das distale Ende des ersten Innenvolumens 9 bewegen zu können, um den gesamten Inhalt des ersten Innenvolumens 9 beim Injizieren herausdrücken zu können. Außerdem ist es vorteilhaft, wenn, wie in Fig. 2 gezeigt, ein distales Ende 27 der Kolbenstange 17 komplementär passend zur Kolbenstangenaufnahme 15 des Stopfens 13 ausgebildet ist. In dem in Fig. 2 gezeigten Ausführungsbeispiel weist das distale Ende 27 der Kolbenstange 17 ein grobes Außengewinde mit wenigen Windungen auf, wobei das Außengewinde genau in das Innengewinde der Kolbenstangenaufnahme 15 des Stopfens 13 passt. Die Kolbenstange 17 kann also in den Stopfen 13 eingedreht werden, bevor der Stopfen 13 axial von der Kolbenstange 17 bewegt wird. Bei ausreichender Elastizität des Stopfens 13 kann das distale Ende 27 der Kolbenstange 17 ggf. einfach axial in die Kolbenstange 15 eingedrückt werden, ohne dass es eines Eindrehens bedarf. Am proximalen Ende 29 der Kolbenstange 17 ist eine Daumendruckfläche 31 ausgebildet, auf welche eine Anwendungsperson mit dem Daumen drücken kann. Der Kragen 24 bildet eine den Spritzenkörper 3 umlaufende distalwärts weisende Fingerzugfläche 33 aus, welche von einer Anwendungsperson mit dem Zeigefinger und Mittelfinger untergriffen werden kann.

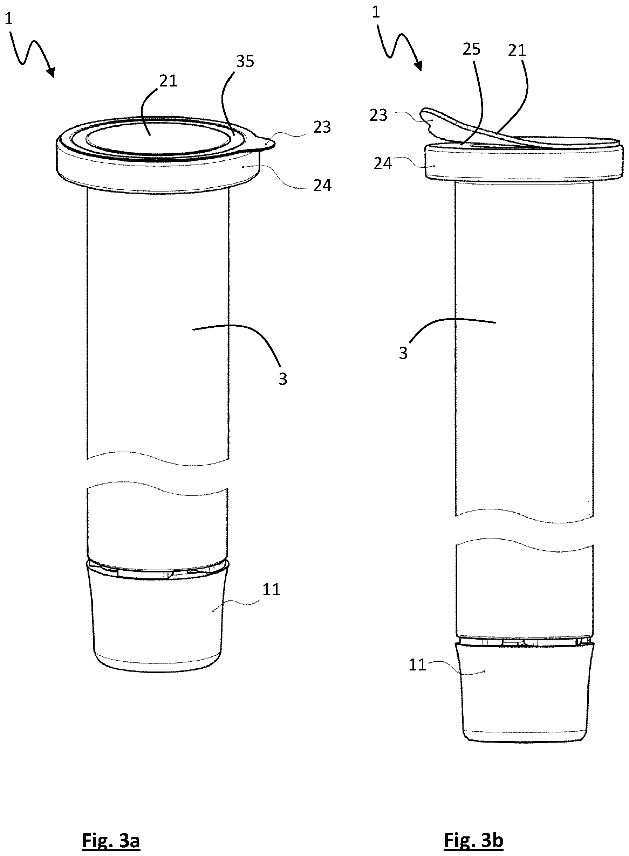

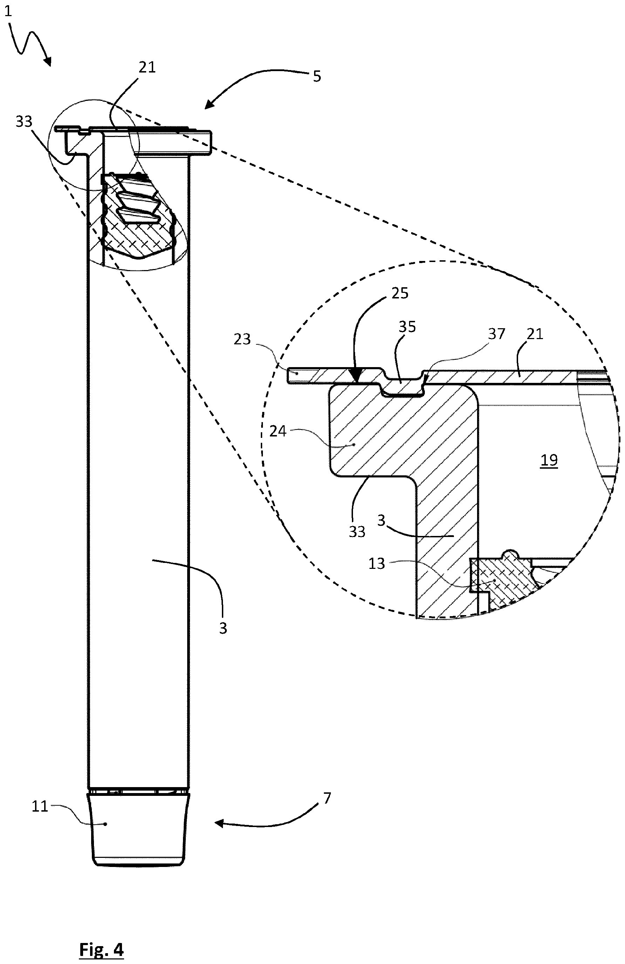

In Fig. 3a,b ist die Möglichkeit verdeutlicht, wie das proximale Dichtmittel 21 mit Hilfe der Griffflasche 23 manuell von der proximalwärts weisenden Ringfläche 25 des Kragens 24 des Spritzenkörpers 3 abgezogen werden kann. Außerdem ist in Fig. 3a erkennbar, dass das proximale Dichtmittel 21 ringförmig umlaufend in die Ringfläche 25 eingeprägt ist. Dies ist in Fig. 4 genauer gezeigt. Durch mechanisches Einstanzen und/oder unter thermoplastischer Verformung ist ein ringförmig umlaufender Abschnitt 35 in eine dazu korrespondierende ringförmig umlaufende Nut 37 in der Ringfläche 25 eingedrückt. Die Nut 37 kann bereits bei der Formung des Spritzenkörpers 3 vorgesehen sein oder beim Einprägen des ringförmigen Abschnitts 35 in die Ringfläche 25 entstehen. Das proximale Dichtmittel 21 wird dabei plastisch verformt und damit zumindest teilweise kraft- und formschlüssig mit dem Kragen 24 des Spritzenkörpers 3 verbunden. Dadurch wird die Verschweißung oder Verklebung verstärkt.

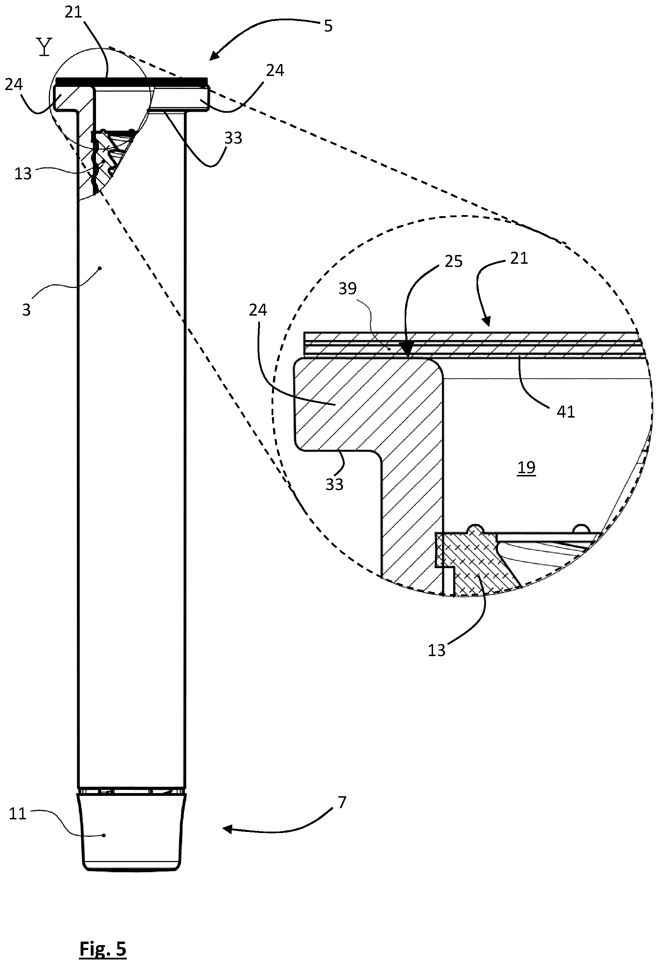

In Fig. 5 ist eine Ausführungsform des proximalen Dichtmittels 21 gezeigt, bei dem das proximale Dichtmittel 21 aus einem Laminat mit mehreren Schichten besteht. Hier ist mindestens eine Schicht des Laminats eine Aluminiumschicht 39, welche eine besonders hohe Barrierewirkung hat. Des Weiteren weist das Laminat des proximalen Dichtmittels 21 eine Schicht 41 aus einem Ccklo-Olefin-Polymer (COP) auf, wobei die COP-Schicht 41 an das zweite Innenvolumen 19 angrenzt, um eine Biokompatibilität mit der vorgefüllten Substanz bereitzustellen, falls diese zumindest teilweise durch den Stopfen 13 in das zweite Innenvolumen 19 diffundiert.

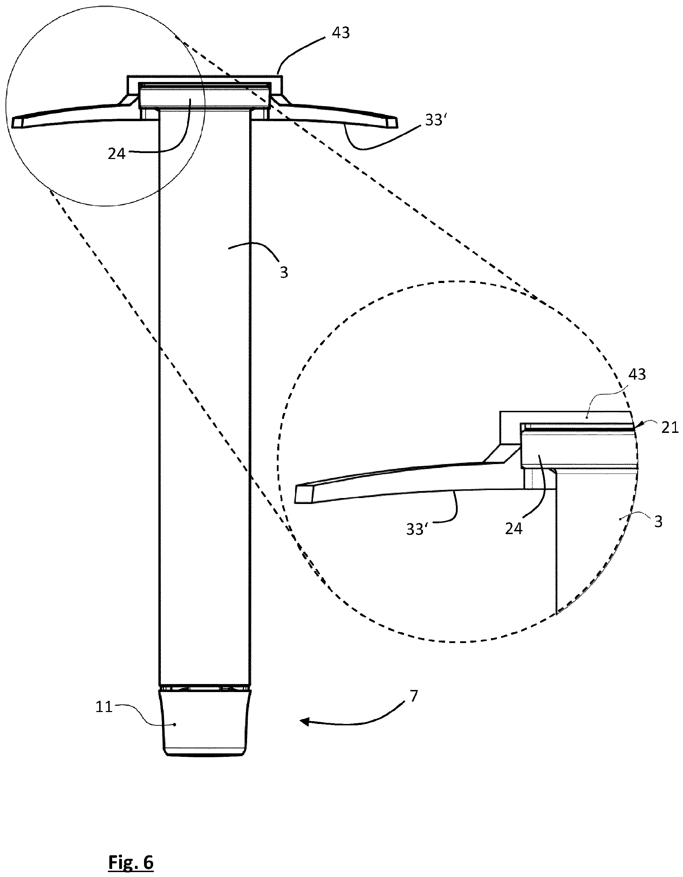

Fig. 6 zeigt ein Ausführungsbeispiel mit einer am proximalen Ende 5 des Spritzenkörpers 3, also am Kragen 24, montierten Griffhilfe 43. Die Griffhilfe 43 dient zum einen dazu, eine größere Fingerzugfläche 33' bereitzustellen als die relativ kleine Fingerzugfläche 33 des Kragens 24 des Spritzenkörpers 3. Die Griffhilfe 43 umgreift hier unter leichter elastischer Verformung den Kragen 24 des Spritzenkörpers 3 und ist dadurch federnd auf die proximalwärts weisende Ringfläche 25 des Kragens 24 vorgespannt und drückt damit das proximale Dichtmittel 21 von oben, also distalwärts, auf die Ringfläche 25. Dies sichert mechanisch die dichtende Verbindung zwischen dem proximalen Dichtmittel 21 und dem Kragen 24.

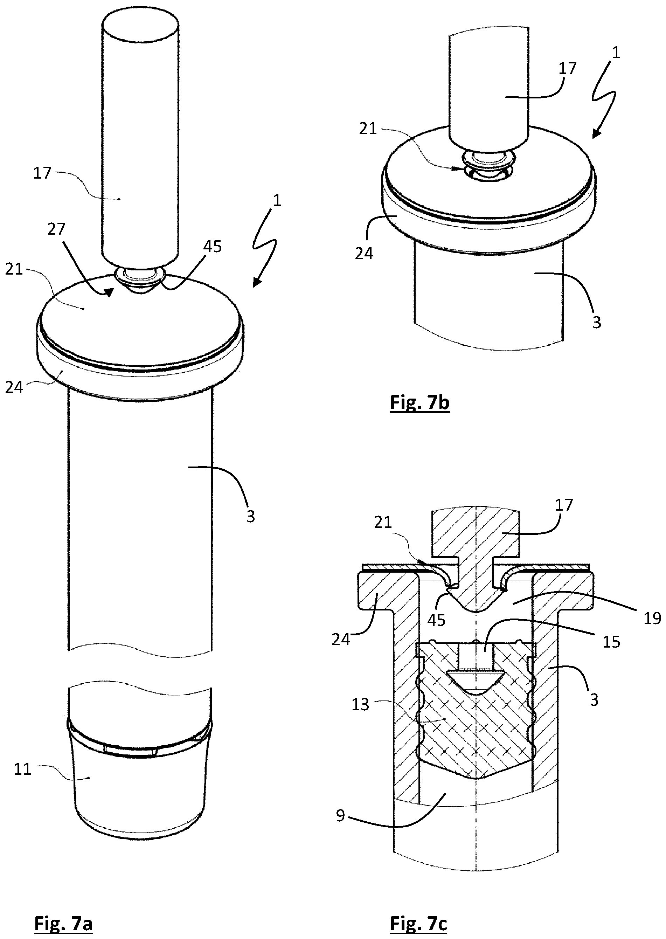

In Fig. 7a-c ist eine andere Ausführungsform der Kolbenstange 17 gezeigt, bei der das distale Ende 27 sich distalwärts verjüngend mit einer lateral umlaufenden Rastnase 45 ausgebildet ist. Durch die distalwärtige Verjüngung bildet die Kolbenstange 17 eine distale Spitze aus, mit welcher die Kolbenstange 17 einfacher durch das proximale Dichtmittel 21 gestoßen werden kann. Die weibliche Kolbenstangenaufnahme 15 des Stopfens 3 ist korrespondierend zum männlichen distalen Ende 27 der Kolbenstange 17 ausgestaltet, sodass die Kolbenstange 17 einfach in den Stopfen 3 unter elastischer Verformung des Stopfens 3 hineingedrückt werden kann, bis die umlaufende Rastnase 45 vom Stopfen 3 hintergriffen wird. Dann ist der Stopfen 3 sicher mit der Kolbenstange 17 verbunden, sodass dann der Stopfen 3 mittels der Kolbenstange 17 für das Injizieren der Substanz distalwärts bewegt werden kann.

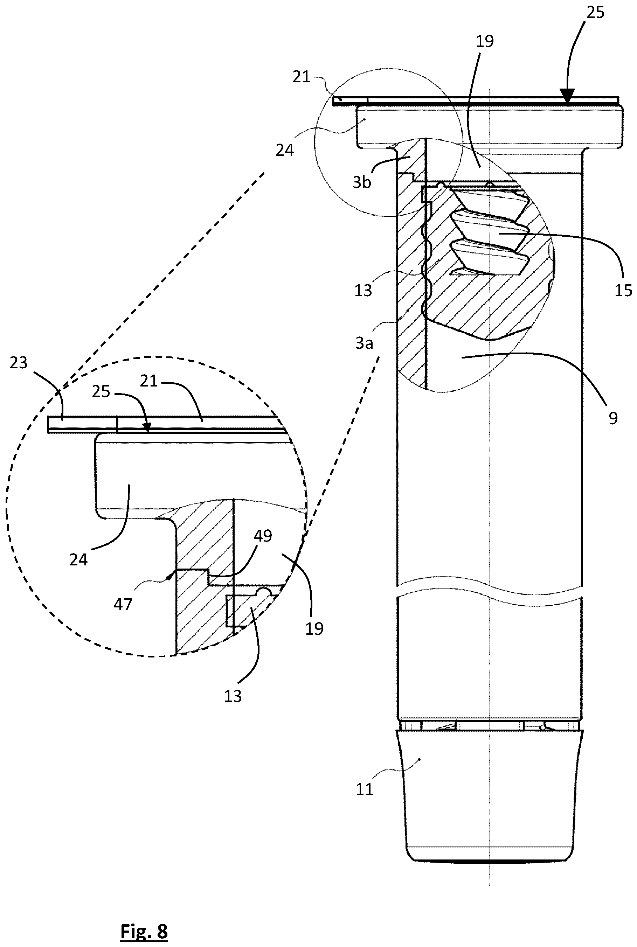

In Fig. 8 ist eine Ausführungsform des Spritzenkörpers 3 gezeigt, der aus zwei miteinander verbundenen Spritzenkörperteilen 3a,b ausgebildet ist. Ein distaler Spritzenkörperteil 3a bildet einen Hauptteil des Spritzenkörpers 3, der das erste Innenvolumen 9 umschließt und auch das distale Ende 7 des Spritzenkörpers 3 definiert. Ein proximaler Spritzenkörperteil 3b bildet einen Kopfteil des Spritzenkörpers 3 und umschließt im Wesentlichen das zweite Innenvolumen 19 und bildet am proximalen Ende 5 des Spritzenkörpers 3 den Kragen 24 mit der proximalwärts weisenden Ringfläche 25 aus. Die beiden Spritzenkörperteile 3a,b sind in dem in Fig. 8 gezeigten Ausführungsbeispiel miteinander verschweißt und/oder verklebt. Eine Fügenaht 47 zwischen den Spritzenkörperteilen 3a,b bildet hier eine umlaufende Fügekante 49 aus, die eine koaxiale Ausrichtung der beiden ineinander gefügten Spritzenkörperteile 3a,b sicherstellt. Bei dieser Ausführungsform kann das proximale Dichtmittel 21 zusammen mit dem proximalen Spritzenkörperteil 3b eine vormontierte Einheit bilden. Der distale Spritzenkörperteil 3a kann mit der Substanz vorbefüllt werden und dann die vormontierte Einheit aus dem proximalen Spritzenkörperteil 3b und dem proximalen Dichtmittel 21 an der Fügenaht 47 miteinander verschweißt und/oder verklebt werden. Dies kann produktionstechnisch vorteilhaft sein, da besondere Umgebungsbedingungen beim Befüllen des Spritzenkörpers 3 herrschen müssen.

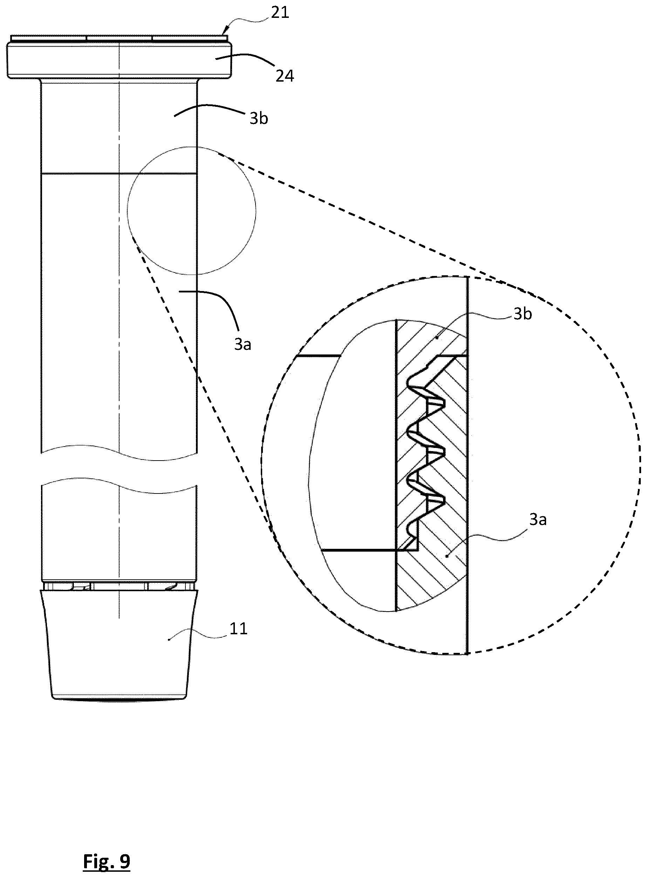

In Fig. 9 ist eine Ausführungsform gezeigt, bei der der Spritzenkörper 3 ebenfalls mehrteilig ausgebildet ist, wobei die Spritzenkörperteile 3a,b miteinander verschraubt sind. Zwischen den Spritzenkörperteilen 3a,b kann eine Dichtung und/oder eine Verklebung zusätzlich zur kraft-und formschlüssigen Verbindung der Verschraubung vorgesehen sein. Eine verklebungsfreie Verschraubung kann beispielsweise vorteilhaft sein, wenn unter den strengen Umgebungsbedingungen am Ort der Befüllung eine Verklebung nicht möglich ist.

Fig. 10a,b zeigen das distale Ende 7 des Applikationsgefäßes 1 genauer. Das distale Ende 7 des Applikationsgefäßes 1 ist hier für die Ausführungsform gemäß Fig. 9 gezeigt, kann allerdings in jeder Ausführungsform Verwendung finden. Das distale Ende 7 des Spritzenkörpers 3 bildet hier einen Anschluss für eine Injektionsnadel oder einen Injektionsschlauch (s. Fig. 11a,b) in Form eines Luer- oder Luer-Lock-Anschlusses 51 aus. Analog zum proximalen Ende 5 des Spritzenkörpers 3 ist hier das distale Ende 7 des Spritzenkörpers 3 mit einem distalen Dichtmittel 53 verschlossen. Das distale Dichtmittel 53 kann genauso ausgestaltet sein wie das proximale Dichtmittel 21, nämlich als manuell abziehbare und/oder durchstoßbare Dichtfolie. Zum manuellen Abziehen kann das distale Dichtmittel 43 analog zum proximalen Dichtmittel 21 eine Griffflasche 55 aufweisen, die lateral übersteht und von einer Anwendungsperson gegriffen werden kann, um das distale Dichtmittel 53 abzuziehen. Zum Durchstoßen des distalen Dichtmittels 53 wird allerdings nicht die Kolbenstange 17 verwendet, sondern eine Injektionsnadel oder ein Injektionsschlauch (s. Fig. 11a,b). Das distale Dichtmittel 53 ist analog zum proximalen Dichtmittel 21 mit dem Spritzenkörper 3 verschweißt und/oder verklebt, um das erste Innenvolumen 9 distalseitig dichtend abzuschließen. Mechanisch gesichert wird das distale Dichtmittel 53 durch die aufgeschraubte Kappe 11, welche vor dem Aufsetzen einer Injektionsnadel oder eines Injektionsschlauchs abgenommen wird.

Fig. 11a zeigt eine beispielhafte Injektionsnadel 57 mit einem weiblichen Luer-Anschluss 59, der an den Luer- oder Luer-Lock-Anschluss 51 des Applikationsgefäßes 1 angeschlossen werden kann. Die Injektionsnadel 57 weist hier eine Kanüle 61 auf, welche sich in den weiblichen Luer-Anschluss 59 der Injektionsnadel 57 erstreckt, sodass die Kanüle 61 das distale Dichtmittel 53 des Applikationsgefäßes 1 durchstoßen kann. Der Injektionsschlauch 63 gemäß Fig. 11b hat einen im Wesentlichen identischen weiblichen Luer-Anschluss 59 wie die Injektionsnadel 57 gemäß Fig. 11a, sodass alternativ auch der Injektionsschlauch 63 an das Applikationsgefäß 1 anschließbar ist. Im weiblichen Luer-Anschluss 59 des Injektionsschlauchs 63 ist ebenfalls ein Kanüle 61 vorgesehen, mit der das distale Dichtmittel 53 durchstoßen werden kann.

### Bezugszeichenliste:

- 1: Applikationsgefäß
- 3: Spritzenkörper
- 3a, b: Spritzenkörperteile
- 5: proximales Ende des Spritzenkörpers
- 7: distales Ende des Spritzenkörpers
- 9: erstes Innenvolumen
- 11: distale Kappe
- 13: Stopfen
- 15: Kolbenstangenaufnahme
- 17: Kolbenstange
- 19: zweites Innenvolumen
- 21: proximales Dichtmittel/Dichtfolie
- 23: Griffflasche
- 24: Kragen
- 25: proximalwärts weisende Ringfläche
- 27: distales Ende der Kolbenstange
- 29: proximales Ende der Kolbenstange
- 31: Daumendruckfläche
- 33, 33': Fingerzugfläche
- 35: ringförmig eingeprägter Abschnitt des proximalen Dichtmittels
- 37: Nut in der proximalwärts weisenden Ringfläche
- 39: Aluminiumschicht
- 41: COP-Schicht
- 43: Griffhilfe
- 45: ringförmig umlaufende Rastnase
- 47: Fügenaht
- 49: Fügekante
- 51: Luer- oder Luer-Lock-Anschluss
- 53: distales Dichtmittel
- 55: Griffflasche
- 57: Injektionsnadel
- 59: weiblicher Luer- oder Luer-Lock-Anschluss
- 61: Kanüle
- 63: Injektionsschlauch

## Patentansprüche

1. Medizinisches Applikationsgefäß (1) mit einem mit einer Substanz zumindest teilweise vorfüllbaren Spritzenkörper (3) zum Injizieren der Substanz in ein Lebewesen, wobei sich der Spritzenkörper (3) von einem proximalen Ende (5) des Spritzenkörpers (3) zu einem distalen Ende (7) des Spritzenkörpers (3) erstreckt und ein erstes Innenvolumen (9) definiert, in welches die Substanz vorfüllbar ist, wobei am distalen Ende (7) des Spritzenkörpers (3) eine Injektionsnadel (57) oder ein Injektionsschlauch (63) angeschlossen oder anschließbar ist, wobei das erste Innenvolumen (9) proximalseitig durch einen auf das distale Ende (7) zu bewegbaren Stopfen (13) begrenzt ist, wobei der Stopfen (13) proximalseitig eine Kolbenstangenaufnahme (15) für eine vom Stopfen (13) separate Kolbenstange (17) aufweist, wobei proximal vom Stopfen (13) ein manuell entfernbares und/oder mittels der Kolbenstange (17) durchstoßbares proximales Dichtmittel (21) angeordnet ist, welches ein axial zwischen dem proximalen Dichtmittel (21) und dem Stopfen (13) ausgebildetes zweites Innenvolumen (19) proximalseitig abdichtet.

2. Medizinisches Applikationsgefäß (1) nach Anspruch 1, wobei der Spritzenkörper (3) zumindest teilweise mit der Substanz vorgefüllt ist, sodass sich die Substanz im ersten Innenvolumen (9) befindet.

3. Medizinisches Applikationsgefäß (1) nach Anspruch 1 oder 2, wobei das Applikationsgefäß (1) eine proximalwärts weisende Ringfläche (25) aufweist, auf welche das proximale Dichtmittel (21) in Form einer manuell abziehbaren und/oder mittels der Kolbenstange (17) durchstoßbaren Dichtfolie dichtend aufgebracht ist.

4. Medizinisches Applikationsgefäß (1) nach einem der vorhergehenden Ansprüche, wobei am distalen Ende (7) des Spritzenkörpers (3) ein Anschluss für die Injektionsnadel (57) oder den Injektionsschlauch (63) in Form eines Luer- oder Luer-Lock-Anschlusses (51) ausgebildet ist.

5. Medizinisches Applikationsgefäß (1) nach einem der vorhergehenden Ansprüche, wobei der Spritzenkörper (3) distalseitig mit einem distalen Dichtmittel (53) verschließbar oder verschlossen ist, wobei das distale Dichtmittel (53) mittels der Injektionsnadel (57) oder des Injektionsschlauchs (63) durchstoßbar ist.

6. Medizinisches Applikationsgefäß (1) nach einem der vorhergehenden Ansprüche, wobei das proximale Dichtmittel (21) distalseitig mit einer Schicht (41) beschichtet ist, die das zweite Innenvolumen (19) proximalseitig begrenzt, wobei die Schicht (41) aus dem gleichen Material ist wie der Spritzenkörper (3), vorzugsweise aus einem Cyclo-Olefin-Polymer (COP) oder einem Polypropylen (PP).

7. Medizinisches Applikationsgefäß (1) nach einem der vorhergehenden Ansprüche, wobei der Spritzenkörper (3) aus Glas ist und/oder einen Kunststoff, vorzugsweise ein Cyclo-Olefin-Polymer (COP) oder Polypropylen (PP), aufweist.

8. Medizinisches Applikationsgefäß (1) nach einem der vorhergehenden Ansprüche, wobei das proximale Dichtmittel (21) mit einer proximalwärts weisenden Ringfläche (25) des Applikationsgefäßes (1) verschweißt und/oder stoffschlüssig verbunden und/oder kraftschlüssig und/oder formschlüssig verbunden ist.

9. Medizinisches Applikationsgefäß (1) nach einem der vorhergehenden Ansprüche, wobei das proximale Dichtmittel (21) mit einer proximalwärts weisenden Ringfläche (25) des Applikationsgefäßes (1) mittels eines Klebers, vorzugsweise eines Schmelzklebers, verklebt ist.

10. Medizinisches Applikationsgefäß (1) nach einem der vorhergehenden Ansprüche, wobei das proximale Dichtmittel (21) aus einer Aluminiumschicht (39) oder einem Laminat mit einer Aluminiumschicht (39) besteht.

11. Medizinisches Applikationsgefäß (1) nach einem der vorhergehenden Ansprüche, wobei das proximale Dichtmittel (21) aus einem Laminat mit einer Schicht (41) aus einem Cyclo-Olefin-Polymer (COP) besteht, wobei die Schicht (41) aus dem Cyclo-Olefin-Polymer (COP) vorzugsweise an das zweite Innenvolumen (19) angrenzt.

12. Medizinisches Applikationsgefäß (1) nach einem der vorhergehenden Ansprüche, wobei das proximale Dichtmittel (21) mittels einer am proximalen Ende (5) des Spritzenkörpers (3) montierten Griffhilfe (43) und/oder eines Backstops unter federnder Vorspannung auf eine proximalwärts weisende Ringfläche (25) des Applikationsgefäßes (1) gedrückt ist.

13. Medizinisches Applikationsgefäß (1) nach einem der vorhergehenden Ansprüche, wobei das axial zwischen dem proximalen Dichtmittel (21) und dem Stopfen (13) angeordnete zweite Innenvolumen (19) mit einem anderen Gas als Luft gefüllt ist.

14. Medizinisches Applikationsgefäß (1) nach einem der vorhergehenden Ansprüche, wobei das proximale Dichtmittel (21) proximalseitig mit Daten über eine Substanzbefüllung und/oder einer einmaligen Produktkennung, UDI, bedruckt ist.

15. Medizinisches Applikationsgefäß (1) nach einem der vorhergehenden Ansprüche, wobei das proximale Dichtmittel (21) als von außen sichtbares Qualitätssiegel dient.

16. Medizinisches Applikationsgefäß (1) nach einem der vorhergehenden Ansprüche, wobei der Stopfen (13) zumindest umfangseitig einen Flüssigsilikonkautschuk (LSR) aufweist, der in gleitfähigem Kontakt mit dem Spritzenkörper (3) steht und vorzugsweise bei Temperaturen oberhalb von -100 °C elastisch ist.

17. Medizinisches Applikationsgefäß (1) nach einem der vorhergehenden Ansprüche, wobei der Stopfen (13) silikonölfrei und gleitbeschichtungsfrei in gleitfähigem Kontakt mit dem Spritzenkörper (3) steht.

18. Medizinisches Applikationsgefäß (1) nach einem der vorhergehenden Ansprüche, wobei der Spritzenkörper (3) einstückig ausgebildet ist und eine proximalseitige Stirnseite des Spritzenkörpers (3) eine proximalwärts weisende Ringfläche (25) bildet, auf welche das proximale Dichtmittel (21) in Form einer manuell abziehbaren und/oder mittels der Kolbenstange (17) durchstoßbaren Dichtfolie dichtend aufgebracht ist.

19. Medizinisches Applikationsgefäß (1) nach einem der Ansprüche 1 bis 17, wobei der Spritzenkörper (3) aus mindestens zwei miteinander verbindbaren oder verbunden Spritzenkörperteilen (3a,b) ausgebildet ist, wobei die Spritzenkörperteile (3a,b) mindestens einen distalen Spritzenkörperteil (3a) und einen proximalen Spritzenkörperteil (3b) aufweisen, wobei das erste Innenvolumen (9) im Wesentlichen vom distalen Spritzenkörperteil (3a) umschlossen ist und das zweite Innenvolumen (19) im Wesentlichen vom proximalen Spritzenkörperteil (3b) umschlossen ist.

20. Medizinisches Applikationsgefäß (1) nach Anspruch 19, wobei der proximale Spritzenkörperteil (3b) mit dem proximalen Dichtmittel (21) eine vormontierte Einheit bildet, bei der das proximale Dichtmittel (21) dichtend auf einer proximalwärts weisenden Ringfläche (25) des proximalen Spritzenkörperteils (3b) sitzt, wobei die vormontierte Einheit mit dem mit der Substanz befüllten distalen Spritzenkörperteil (3a) verbindbar oder verbunden ist.

21. Medizinisches Applikationsgefäß (1) nach Anspruch 19 oder 20, wobei der distale Spritzenkörperteil (3a) axial länger ist als der proximale Spritzenkörperteil (3b), vorzugsweise um ein Vielfaches.
